# FASCICULE DE BREVET EUROPEEN

(11) **EP 3 996 785 B1**
(45) Date de publication et mention de la délivrance du brevet: **26.04.2023**
(21) Numéro de dépôt: 20820240.8
(22) Date de dépôt: 09.07.2020
(51) Int. Cl.: A61M 15/08, A61M 11/02, B05B 11/06

(54) **DISPOSITIF DE DISTRIBUTION NASALE DE POUDRE**
VORRICHTUNG ZUR NASALEN VERABREICHUNG EINES PULVERS
NASAL DELIVERY DEVICE FOR A POWDER

(30) Priorité: 10.07.2019 FR 1907764
(43) Date de publication de la demande: 18.05.2022
(73) Titulaire: APTAR France SAS, 27110 Le Neubourg (FR)
(72) Inventeur: PETIT, Ludovic, 27110 Vitot (FR); POULLAIN, Franck, 27400 La Haye Malherbe (FR)
(74) Mandataire: CAPRI
(86) Numéro de dépôt international: PCT/FR2020/051225
(87) Numéro de publication internationale: WO 2021/005308

(56) Documents cités:
- WO-A1-91/12895
- WO-A1-2015/001281
- WO-A1-2017/118827
- WO-A2-2018/051371
- FR-A1- 2 684 304
- US-A- 5 284 132

## Description

La présente invention concerne un dispositif de distribution nasale de poudre.

Les dispositifs de distribution nasale de poudre sont bien connus. Ils comportent généralement un réservoir contenant une ou plusieurs doses de poudre, des moyens de distribution, et une tête de distribution nasale destinée à être insérée dans une narine d'un utilisateur, ladite tête de distribution nasale comportant un orifice de distribution. Les moyens de distribution comportent généralement une chasse d'air. Lorsque le dispositif de distribution est actionné, une dose de poudre est distribuée dans une narine de l'utilisateur.

Un inconvénient avec ces dispositifs de l'art antérieur concerne la fiabilité du dispositif, notamment avant et lors de l'actionnement. Ainsi, pour maintenir le dispositif dans l'état non actionné pendant le transport et/ou en cas de chute accidentelle, on prévoit généralement des ponts sécables entre deux parties mobiles l'une par rapport à l'autre lors de l'actionnement. Ces ponts sécables présentent plusieurs inconvénients: ils sont difficiles à mouler, et la force de rupture est difficilement maitrisable. De plus, en raison de leur état statique avant rupture, ils sont susceptibles de se casser en cas de chute accidentelle du dispositif.

Les documents WO9946055, WO0245866, WO2015001281, WO2017118827, WO2018051371, WO9112895, US5284132 et FR2684304 décrivent des dispositifs de l'état de la technique.

La présente invention a pour but de fournir un dispositif de distribution nasale de poudre qui ne reproduit pas les inconvénients susmentionnés.

La présente invention a aussi pour but de fournir un dispositif de distribution nasale de poudre qui améliore la fiabilité du dispositif avant et pendant l'actionnement.

La présente invention a également pour but de fournir un dispositif de distribution nasale de poudre qui est simple et peu coûteux à fabriquer et à assembler.

La présente invention a donc pour objet un dispositif de distribution nasale de poudre comportant un réservoir contenant au moins une dose de poudre, une tête de distribution nasale destinée à être insérée dans une narine d'un utilisateur, ladite tête de distribution nasale comportant un orifice de distribution, et une chasse d'air générant, lors de l'actionnement dudit dispositif de distribution nasale de poudre, un écoulement d'air comprimé pour distribuer une dose de poudre dans ladite narine à travers ledit orifice de distribution, ladite chasse d'air comprenant une chambre d'air, formée par un cylindre axial creux, et un piston qui lors de l'actionnement du dispositif coulisse de manière étanche dans ladite chambre d'air pour comprimer l'air contenu dans ladite chambre d'air, ledit cylindre axial creux comportant un épaulement radial qui relie une première partie de cylindre de plus grand diamètre et une seconde partie de cylindre de plus petit diamètre, ledit piston étant disposé avant actionnement au niveau de ladite première partie de cylindre, et lors de l'actionnement, ledit piston coopère de manière étanche avec ladite seconde partie de cylindre, pour comprimer l'air contenu dans ladite chambre d'air et ainsi générer l'écoulement d'air comprimé, ledit épaulement radial définissant dans ledit cylindre axial creux un profil que ledit piston doit surmonter en début d'actionnement, ledit piston comportant une lèvre supérieure et une lèvre inférieure, une jupe étant disposée autour dudit cylindre axial creux, ladite jupe comportant à proximité de son bord axialement inférieur une bride inférieure, radialement saillante vers l'intérieur, ladite jupe comportant à proximité de ladite bride inférieure au moins un profil radialement saillant vers l'intérieur coopérant avant actionnement avec ladite lèvre inférieure dudit piston.

Avantageusement, avant actionnement, ledit piston est déplaçable entre deux positions non actionnées, une position de transport inférieure, dans laquelle ladite lèvre inférieure dudit piston est en butée contre ladite bride inférieure de ladite jupe, et une position de transport supérieure, dans laquelle ladite lèvre supérieure dudit piston est en butée contre ledit épaulement radial dudit cylindre creux.

Avantageusement, lesdits profils sont formés par une ou plusieurs nervures axiales réparties sur la périphérie de ladite jupe.

Avantageusement, ladite chambre d'air est ouverte sur l'atmosphère avant actionnement.

Avantageusement, ledit réservoir contient une seule dose de poudre, distribuée lors d'un seul actionnement dudit dispositif de distribution nasale de poudre.

Ces caractéristiques et avantages et d'autres apparaîtront plus clairement au cours de la description détaillée suivante, faite en référence aux dessins joints, donnés à titre d'exemples non limitatifs, et sur lesquels :
les figures 1 et 2 sont des vues schématiques en section transversale d'un dispositif de distribution nasale de poudre selon un mode de réalisation avantageux, respectivement en position transport inférieure et supérieure,
la figure 3 est une vue similaire à celles des figures 1 et 2, en position de repos, avant actionnement,
la figure 4 est une vue similaire à celle de la figure 3, en début d'actionnement,

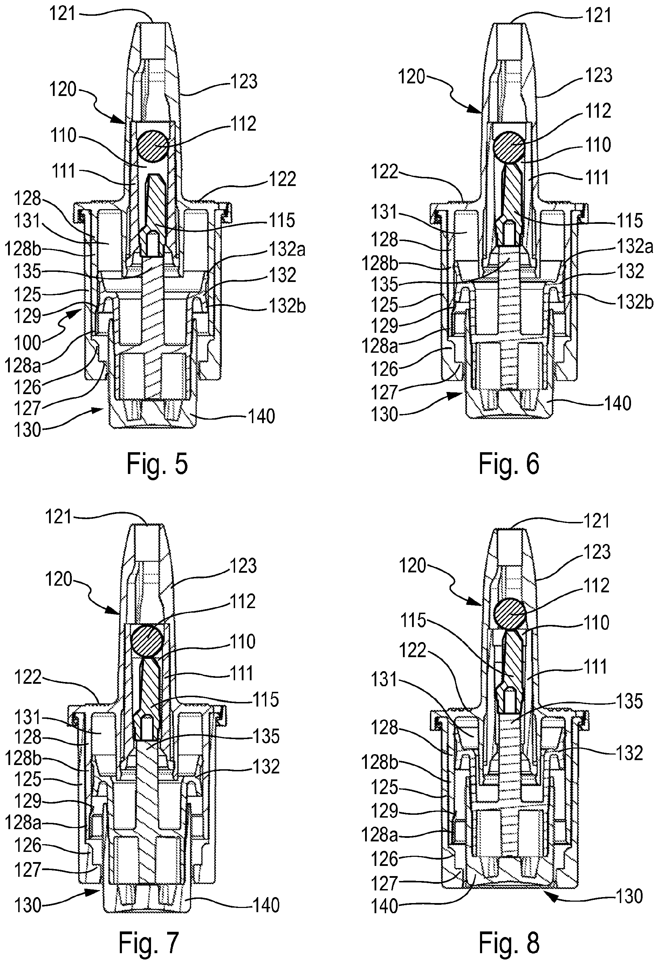

les figures 5 à 7 sont des vues similaires à celle de la figure 3, montrant différentes positions en cours d'actionnement, et
la figure 8 est une vue similaire à celle de la figure 3, en fin d'actionnement.

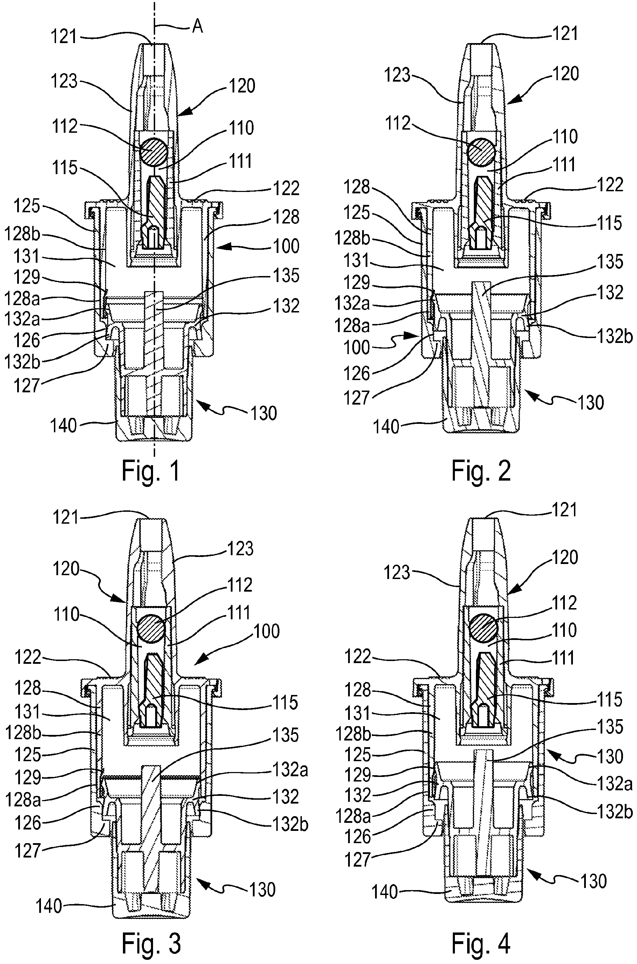

Dans la description, les termes "axial" et "radial" se réfèrent à l'axe longitudinale A du dispositif représenté sur la figures 1. Les termes "proximal" et "distal" se réfèrent à l'orifice de distribution dudit dispositif. Les termes "haut", "bas", "supérieur" et "inférieur" se réfèrent à la position droite du dispositif représentée sur les dessins.

L'invention s'applique plus particulièrement à des dispositifs du type unidose poudre tel que celui représenté sur les figures. Bien entendu, d'autres types de dispositifs de distribution nasale de poudre sont aussi envisageables.

Le dispositif 100 représenté sur les figures comporte un réservoir 110 contenant une seule dose de poudre. Des dispositifs avec un réservoir contenant plus d'une dose sont possibles. De même, des dispositifs comportant plusieurs réservoirs contenant chacune une seule dose sont aussi possibles.

Une tête de distribution nasale 120 est assemblée sur ledit réservoir 110, ladite tête étant destinée à être insérée dans une narine d'un utilisateur. Ladite tête de distribution nasale comporte un orifice de distribution 121. La tête de distribution 120 comporte avantageusement un repose-doigt 122 s'étendant radialement pour faciliter l'actionnement. Un manchon creux 123 s'étend axialement vers le haut à partir dudit repose-doigt 122 et se termine au niveau dudit orifice de distribution 121. De préférence, ce manchon creux 123 est de dimension radiale réduite pour pouvoir être inséré dans une narine au moment de l'actionnement. Du côté opposé du repose-doigt 122, une jupe 125 s'étend axialement vers le bas à partir dudit repose-doigt 122. Ladite jupe 125 comporte à proximité de son bord axialement inférieur une bride inférieure 127, radialement saillante vers l'intérieur.

Le dispositif 100 comporte en outre une chasse d'air 130 générant, lors de l'actionnement dudit dispositif 100, un écoulement d'air comprimé pour distribuer une dose de poudre dans ladite narine à travers ledit orifice de distribution 121. Ladite chasse d'air comprend une chambre d'air 131 et un piston 132 coulissant de manière étanche dans ladite chambre d'air 131 pour comprimer l'air contenu dans ladite chambre d'air 131 et ainsi générer ledit écoulement d'air comprimé. Le piston 132 comporte une lèvre supérieure 132a et une lèvre inférieure 132b. La chambre d'air 131 est formée par un cylindre axial creux 128 qui est solidaire, de préférence monobloc, avec le repose-doigt 122 de la tête de distribution 120. Le côté inférieur dudit cylindre creux 128 est ouvert et obturé par ledit piston 132. La jupe 125 est disposée autour dudit cylindre creux 128, et peut notamment être formée par un manchon creux fixé, par exemple encliqueté, dans le repose-doigt 122 de la tête de distribution 120. La bride inférieure 127 de la jupe 125 forme une butée inférieure pour le piston 132, notamment pour sa lèvre inférieure 132b, comme visible sur la figure 1.

Le piston 132 est de préférence solidaire d'un organe d'actionnement 140 sur lequel l'utilisateur va appuyer lors de l'actionnement pour déplacer le piston 132 dans la chambre d'air 131.

Dans l'exemple représenté sur les figures, le réservoir 110 est formé par un tube creux 111 ouvert à ses deux extrémités axiales, et fermé à son extrémité proximale par un élément de fermeture 112, tel qu'une bille, et fermé à son extrémité distale par un insert 115. Cet insert 115 comporte une extension axiale formant tige, et peut, lors de l'actionnement, coulisser dans ledit tube creux 111 pour chasser ledit élément de fermeture 112 hors de sa position de fermeture. Dans cet exemple, le piston 132 de la chasse d'air 130 est solidaire d'une projection axiale 135 qui s'étend en direction proximale, et qui, lors de l'actionnement, va se déplacer ensemble avec le piston 132 lors de la compression de l'air contenu dans la chambre d'air 131. Lorsque ladite projection 135 du piston 132 vient en contact avec ledit insert 115 du réservoir 110, une continuation du déplacement du piston 132 va provoquer le coulissement dudit insert 115 dans ledit tube creux 111 hors de sa position de fermeture. Ledit insert 115 va d'une part ouvrir le passage entre la chasse d'air 130 et le réservoir 110 et d'autre part provoquer l'expulsion de l'élément de fermeture 112. Ainsi, l'air comprimé dans la chambre d'air 131 va s'écouler dans ledit réservoir et entrainer la dose de poudre hors dudit réservoir en direction dudit orifice de distribution 121. Les documents WO9946055, WO0245866, WO2015001281 et WO2017118827 décrivent des dispositifs de ce type. Bien entendu, d'autres types de dispositifs sont aussi possibles.

Selon l'invention, le cylindre axial creux 128 qui forme la chambre d'air 131 comporte un épaulement radial 129. Cet épaulement radial 129 définit dans la paroi latérale dudit cylindre 128 un profil que le piston 132, notamment sa lèvre supérieure 132a, doit surmonter en début d'actionnement. Cet épaulement radial 129 forme donc une résistance à l'actionnement que l'utilisateur doit surmonter en début d'actionnement, et donc une sécurité transport pour éviter un actionnement accidentel du dispositif.

Ledit épaulement radial 129 relie une première partie de cylindre 128a de plus grand diamètre et une seconde partie de cylindre 128b de plus petit diamètre. Avant actionnement, le piston 132 est disposé au niveau de la première partie de cylindre 128a, de sorte qu'il ne coopère pas nécessairement de manière étanche avec le cylindre 128. Ainsi, au repos, la chambre d'air 131 est avantageusement ouverte sur l'atmosphère. Lors de l'actionnement, le piston 132 coopère de manière étanche avec la seconde partie de cylindre 128b, pour comprimer l'air contenu dans la chambre d'air 131 et ainsi générer l'écoulement d'air comprimé.

Comme visible sur les figures 1 et 2, le piston 132 peut avant actionnement se déplacer entre deux positions non actionnées, une position dite de transport inférieure, visible sur la figure 1, et une position de transport supérieure, visible sur la figure 2. Dans la position de transport inférieure, la lèvre inférieure 132b du piston 132 est en butée contre la bride inférieure 127 de la jupe 125. Dans la position de transport supérieure, la lèvre supérieure 132a du piston 132 est en butée contre l'épaulement radial 129 du cylindre 128. Ainsi, au repos, le piston 132 est dans une position quelconque situées entre les deux positions de transport, et en cas de chute accidentelle, le dispositif n'est donc pas statique, comme cela serait le cas si le piston était fixé à la jupe par des ponts sécables. Alors que de tels ponts sécables pourraient se casser en cas de chute, par exemple lors d'un drop-test, il n'en est rien avec l'épaulement radial 129 de la présente invention. La sécurité transport et la résistance aux chutes sont donc améliorées avec la présente invention.

Ladite jupe 125, dans la partie inférieure de sa paroi latérale, à proximité de la bride inférieure 127, comporte au moins un profil radialement saillant vers l'intérieur 126. Ces profils 126 peuvent avantageusement être formés par une ou plusieurs nervures axiales, par exemple trois ou quatre nervures réparties sur la périphérie de la jupe 125. Ces profils 126 coopèrent avec la lèvre inférieure 132b du piston 132 avant actionnement, c'est-à-dire quand le piston 132 est situé entre ses positions transport inférieure et supérieure, comme visible sur les figures 1 à 3. Ce n'est qu'au début de l'actionnement, visible sur la figure 4, que la lèvre inférieure 132b se dégage desdits profils 126. La présence de ces profils 126 renforce la coopération par frottement entre le piston 132 et la jupe 125, et participe donc à la sécurité transport.

Une combinaison de l'épaulement radial 129 du cylindre axial creux 128 et des profils 126 de la jupe 125 procure un résultat optimal concernant la sécurité transport, tout en permettant de prédéterminer avec plus de précision le seuil de force nécessaire pour l'utilisateur pour actionner le dispositif, en comparaison avec des ponts sécables par exemple. De plus, aussi bien l'épaulement radial 129 que les profils 126 sont moins compliqués à mouler que des ponts sécables, et la présente invention permet donc de simplifier la fabrication et l'assemblage du dispositif.

La présente invention a été décrite en référence à un mode de réalisation avantageux, mais il est entendu qu'un homme du métier peut y apporter toutes modifications, sans sortir du cadre de la présente invention tel que défini par les revendications annexées.

## Revendications

1. Dispositif de distribution nasale de poudre (100) comportant un réservoir (110) contenant au moins une dose de poudre, une tête de distribution nasale (120) destinée à être insérée dans une narine d'un utilisateur, ladite tête de distribution nasale comportant un orifice de distribution (121), et une chasse d'air (130) générant, lors de l'actionnement dudit dispositif de distribution nasale de poudre (100), un écoulement d'air comprimé pour distribuer une dose de poudre dans ladite narine à travers ledit orifice de distribution (121), ladite chasse d'air comprenant une chambre d'air (131), formée par un cylindre axial creux (128), et un piston (132) qui lors de l'actionnement du dispositif coulisse de manière étanche dans ladite chambre d'air (131) pour comprimer l'air contenu dans ladite chambre d'air (131), ledit cylindre axial creux (128) comporte un épaulement radial (129) qui relie une première partie de cylindre (128a) de plus grand diamètre et une seconde partie de cylindre (128b) de plus petit diamètre, ledit piston (132) étant disposé avant actionnement au niveau de ladite première partie de cylindre (128a), et lors de l'actionnement, ledit piston (132) coopère de manière étanche avec ladite seconde partie de cylindre (128b), pour comprimer l'air contenu dans ladite chambre d'air (131) et ainsi générer l'écoulement d'air comprimé, ledit épaulement radial (129) définissant dans ledit cylindre axial creux (128) un profil que ledit piston (132) doit surmonter en début d'actionnement, ledit piston (132) comportant une lèvre supérieure (132a) et une lèvre inférieure (132b), une jupe (125) étant disposée autour dudit cylindre axial creux (128), ladite jupe (125) comportant à proximité de son bord axialement inférieur une bride inférieure (127), radialement saillante vers l'intérieur, **caractérisé en ce que** ladite jupe (125) comporte à proximité de ladite bride inférieure (127) au moins un profil radialement saillant vers l'intérieur (126) coopérant avant actionnement avec ladite lèvre inférieure (132b) dudit piston (132).

2. Dispositif selon la revendication 1, dans lequel, avant actionnement, ledit piston (132) est déplaçable entre deux positions non actionnées, une position de transport inférieure, dans laquelle ladite lèvre inférieure (132b) dudit piston (132) est en butée contre ladite bride inférieure (127) de ladite jupe (125), et une position de transport supérieure, dans laquelle ladite lèvre supérieure (132a) dudit piston (132) est en butée contre ledit épaulement radial (129) dudit cylindre creux (128).

3. Dispositif selon la revendication 1 ou 2, dans lequel lesdits profils (126) sont formés par une ou plusieurs nervures axiales réparties sur la périphérie de ladite jupe (125).

4. Dispositif selon l'une quelconque des revendications précédentes, dans lequel ladite chambre d'air (131) est ouverte sur l'atmosphère avant actionnement.

5. Dispositif selon l'une quelconque des revendications précédentes, dans lequel ledit réservoir (110) contient une seule dose de poudre, distribuée lors d'un seul actionnement dudit dispositif de distribution nasale de poudre (100).

## Patentansprüche

1. Vorrichtung zur nasalen Verabreichung eines Pulvers (100) aufweisend einen Vorratsbehälter (110), der wenigstens eine Pulverdosis enthält, einen Kopf zur nasalen Verabreichung (120) zur Einführung in ein Nasenloch eines Benutzers, wobei der Kopf zur nasalen Verabreichung eine Abgabeöffnung (121) und eine Luftausstoßeinrichtung (130) aufweist, die bei der Betätigung der Vorrichtung zur nasalen Verabreichung eines Pulvers (100) einen Druckluftstrom erzeugt, um eine Pulverdosis durch die Abgabeöffnung (121) in das Nasenloch abzugeben, wobei die Luftausstoßeinrichtung eine Luftkammer (131), die von einem axialen Hohlzylinder (128) gebildet ist, und einen Kolben (132) umfasst, der bei der Betätigung der Vorrichtung dicht in der Luftkammer (131) gleitet, um die in der Luftkammer (131) enthaltene Luft zu komprimieren, der axiale Hohlzylinder (128) einen radialen Absatz (129) aufweist, der einen ersten Zylinderabschnitt (128a) größeren Durchmessers und einen zweiten Zylinderabschnitt (128b) kleineren Durchmessers verbindet, wobei der Kolben (132) vor Betätigung am ersten Zylinderabschnitt (128a) angeordnet ist und der Kolben (132) bei der Betätigung dicht mit dem zweiten Zylinderabschnitt (128b) zusammenwirkt, um die in der Luftkammer (131) enthaltene Luft zu komprimieren und so den Druckluftstrom zu erzeugen, wobei der radiale Absatz (129) in dem axialen Hohlzylinder (128) ein Profil definiert, das der Kolben (132) zu Betätigungsbeginn überwinden muss, wobei der Kolben (132) eine obere Lippe (132a) und eine untere Lippe (132b) aufweist, wobei ein Mantel (125) um den axialen Hohlzylinder (128) herum angeordnet ist, wobei der Mantel (125) in der Nähe seines axial unteren Randes einen unteren Flansch (127) aufweist, der radial nach innen hervorsteht, **dadurch gekennzeichnet, dass** der Mantel (125) in der Nähe des unteren Flansches (127) wenigstens ein radial nach innen hervorstehendes Profil (126) aufweist, das vor Betätigung mit der unteren Lippe (132b) des Kolbens (132) zusammenwirkt.

2. Vorrichtung nach Anspruch 1, wobei vor Betätigung der Kolben (132) zwischen zwei nicht betätigten Positionen bewegbar ist, einer unteren Transportposition, in der die untere Lippe (132b) des Kolbens (132) an dem unteren Flansch (127) des Mantels (125) anliegt, und einer oberen Transportposition, in der die obere Lippe (132a) des Kolbens (132) an dem radialen Absatz (129) des Hohlzylinders (128) anliegt.

3. Vorrichtung nach Anspruch 1 oder 2, wobei die Profile (126) von einer oder mehreren axialen Rippen gebildet sind, die auf dem Umfang des Mantels (125) verteilt sind.

4. Vorrichtung nach einem der vorhergehenden Ansprüche, wobei die Luftkammer (131) vor Betätigung zur Atmosphäre hin offen ist.

5. Vorrichtung nach einem der vorhergehenden Ansprüche, wobei der Vorratsbehälter (110) eine einzelne Pulverdosis enthält, die bei einer einzelnen Betätigung der Vorrichtung zur nasalen Verabreichung eines Pulvers (100) abgegeben wird.

## Claims

1. A nasal powder delivery device (100) comprising a reservoir (110) containing at least one dose of powder, a nasal delivery head (120) intended to be inserted in a nostril of a user, said nasal delivery head comprising a delivery aperture (121), and an air discharge system (130) generating, upon actuation of said nasal powder delivery device (100), a flow of compressed air to deliver a dose of powder in said nostril through said delivery aperture (121), said air discharge system comprising an air chamber (131), formed by a hollow axial cylinder (128) and a piston (132) which, upon actuation of the device, sealingly slides in said air chamber (131) to compress the air contained in said air chamber (131), said hollow axial cylinder (128) comprises a radial shoulder (129) which connects a first cylinder portion (128a) of larger diameter and a second cylinder portion (128b) of smaller diameter, said piston (132) being arranged before actuation at said first cylinder portion (128a), and upon actuation, said piston (132) sealingly cooperates with said second cylinder portion (128b), to compress the air contained in said air chamber (131), and thus generate the flow of compressed air, said radial shoulder (129) defining in said hollow axial cylinder (128), a profile that said piston (132) must overcome at the start of actuation, said piston (132) comprising an upper lip (132a) and a lower lip (132b), a skirt (125) being arranged around said hollow axial cylinder (128), said skirt (125) comprising, in the vicinity of its axially lower edge, a lower flange (127), which radially projects inwards, **characterised in that** said skirt (125) comprises, in the vicinity of said lower flange (127), at least one profile, which radially projects inwards (126) cooperating before actuation with said lower lip (132b) of said piston (132).

2. The device according to claim 1, wherein, before actuation, said piston (132) can be moved between two non-actuated positions, a lower transport position, in which said lower lip (132b) of said piston (132) is abutted against said lower flange (127) of said skirt (125), and an upper transport portion, in which said upper lip (132a) of said piston (132) is abutted against said radial shoulder (129) of said hollow cylinder (128).

3. The device according to claim 1 or 2, wherein said profiles (126) are formed by one or more axial ridges distributed over the periphery of said skirt (125).

4. The device according to any one of the preceding claims, wherein said air chamber (131) is open to the atmosphere before actuation.

5. The device according to any one of the preceding claims, wherein said reservoir (110) contains one single dose of powder, distributed upon one single actuation of said nasal powder delivery device (100).
